Europäisches Patentamt

European Patent Office

Office européen des brevets

(19)

(11) Publication number: **0 003 914**
**B1**

(12) **EUROPEAN PATENT SPECIFICATION**

(45) Date of publication of patent specification: **02.05.84**

(21) Application number: **79300293.2**

(22) Date of filing: **26.02.79**

(51) Int. Cl.³: **A 61 M  1/03,**
**B 01 D  13/00, B 01 D  15/00**

(54) Dialysis composition.

(30) Priority: **27.02.78 US 881684**

(43) Date of publication of application:
**05.09.79 Bulletin 79/18**

(45) Publication of the grant of the patent:
**02.05.84 Bulletin 84/18**

(84) Designated Contracting States:
**DE GB IT NL SE**

(56) References cited:
**DE - A - 2 512 212**
**DE - A - 2 558 363**
**US - A - 3 463 728**
**US - A - 3 669 878**
**US - A - 4 071 444**

(73) Proprietor: **PURDUE RESEARCH FOUNDATION**
**Hovde Hall Purdue University**
**West Lafayette Indiana 47907 (US)**

(72) Inventor: **Ash, Stephen Richard**
**2500 N. County Road**
**400 East Lafayette Indiana (US)**

(74) Representative: **Bannerman, David Gardner et al,**
**Withers & Rogers 4 Dyer's Buildings Holborn**
**London, EC1N 2JT (GB)**

Courier Press, Leamington Spa, England.

## Dialysis composition

This invention relates to a dialysis composition comprising a surface adsorptive agent, an enzyme, a suspending agent and cation exchange agent, and more particularly, relates to a material for use in an artificial kidney to remove uremic substances during hemodialysis.

A. When the kidneys fail to provide excretory function for the body, most of the other organs of the body soon fail. The numerous symptoms which ensue are termed "uremia" and include bleeding, nausea and vomiting, cardiac arrhythmias, and coma. The severity of these symptoms is proportional to the retention of several known chemical compounds, and the improvement during treatment with the artificial kidney is proportional to the removal of these substances.

Some known substances which must be removed are: $Na^+$, $H^+$, $K^+$, $Mg^+$, urea, creatinine, $PO_4^=$, and a variety of "middle molecules", or polypeptides, phenols, guanidines, amines, etc. Urea is the most abundant of these substances; 15—30 grams is produced daily by the adult human. The amount of substance removed, or the mass transfer, must also occur at a relatively low concentration of the substance in the blood; in other words, the "mass transfer coefficient" must be high. In biologic terms, "mass transfer coefficient" is similar to clearance, or the amount of substance removed per unit time, divided by the concentration of the substance in the blood; the higher the clearance, the lower the blood concentration of the above substances, and the less the uremic symptoms.

The artificial kidney treatment procedure generally used today is similar to that developed by Kolff in the early 1940's. Blood is removed from the body, and propelled through a closed system of membranes, which are semipermeable, and returned to the body. These membranes allow the passage of small molecules, and retain larger molecules such as proteins, as well as cellular blood elements. The uremic substances, being small molecules, will pass through the membranes, as long as the concentration of these substances is kept low on the outside of the membranes. This process is called "dialysis".

In the original artificial kidney, and in most of those in use today, the concentration of uremic substances outside the membranes is kept low by dilution in a large volume of water called "dialysate". A volume of water of 120—200 l is used per dialysis treatment. The water is prepared in a bath, or continually, and must be treated to remove trace elements, mixed with a concentration of electrolytes and glucose, and warmed to blood temperature. Electrolytes which must be added to the dialysate to prevent excessive removal are $Ca^{++}$, $Mg^{++}$, $K^+$, $Na^+$, $HCO_3^-$ (in the form of acetate), and $Cl^-$. Calcium should be in a concentration to cause addition of calcium to the patient's blood, as the total body calcium of kidney failure patients is often low, and the stimulation of parathyroid hormone by low calcium is detrimental to the patient's health.

Besides a large volume of water, present artificial kidneys require a rapid velocity of water near the membranes to keep the concentration of uremic substances low. This is accomplished by "re-circulation" of water at a fast rate, 10 l per minute, through the membranes in a "coil" dialyzer. In a "plate" or "hollow fiber" dialyzer a slower flow of dialysate may be used because the dialysate is restricted to a narrow chamber next to the membranes; this also results in a rapid water velocity near the membranes. The removal of water from the blood requires that pressure be higher on the blood side of the membranes, than on the dialysate side of the membranes; a positive pressure of 100—300 mm Hg is created in "coil" dialyzers, and a negative pressure is developed on the dialysate side of "plate" or "hollow fiber" dialysers.

The usual artificial kidney machine must thus include: access to a large volume of water, mechanism for addition of vital substances to matter, a blood pump, dialysis pump, membrane package, water heater, and often a recirculating pump for water near the membranes. In addition, monitoring equipment is used for blood and dialysate pressure, dialysate temperature, and concentration of electrolytes (vital) in the dialysate. Water treatment equipment is needed for removal of toxic materials from the large volume of water. The total amount of equipment is complex, expensive and immobile. Artificial kidney machines are shown, by way of example, in U.S. Patent Numbers 3,352,422; 3,362,540; 3,570,672; 3,682,817; 3,864,248; and French 2,263,017.

A different approach to hemodialysis is to regenerate dialysis fluid, for re-utilization. In a system, developed utilizing this approach, the dialysis fluid is removed from a bath near the membranes, forced through a column of absorbing compounds to remove uremic substances, and returned to the dialysate bath. The adsorbing compounds are those which have been utilized for decades for removal of uremic substances:

a. Charcoal, first described by Yatzidis in 1964 as having properties of adsorption of most uremic substances (creatinine, and "middle molecules").

b. Urease, known for many years as the major enzyme for degradation of urea in plants, bacteria and animals. It splits urea to $NH_4$ and $CO_3^=$.

c. Zirconium phosphate, a standard cation exchanger which has a high affinity for divalent cations and a lower affinity for monovalent cations, as described by Maeck, in 1963. Zirconium phosphate in at least one prior art system is a gel, and is "loaded" with 90% $H^+$, 10% $Na^+$.

d. Zirconium oxide, anion exchange resin for removal of phosphate and fluoride.

In the system as presently developed, these materials are arranged in sequential layers, in a column which weighs approximately 12 pounds, and which has maximal capacity of approximately 30 grams urea removal.

Ideally, the use of regeneration of dialysate would result in a simpler, and more mobile dialysis system. However, presently developed systems are instead more complex and not easily portable, weighing approximately 44 lbs (20 kg). In addition, several problems accompany its use:

a. Sequential layering of adsorbent compounds does not allow favorable interactions of certain elements. Urease, for example, is inhibited by its own products, $NH_4^+$ and $CO_3^=$. Zirconium phosphate, which adsorbs $NH_4^+$ and buffers $CO_3^=$, is not present in the urease layer. As a result, large amounts of urease are needed to overcome inhibition.

b. Adsorbent compounds must be carefully sized to prevent packing of the column by the dialysate flow. Nonetheless, at flow rates over 300 ml/minute, packing does occur, and resistance through the column increases. Thus, the regenerated dialysate flow rate is less than that of dialysate in other artificial kidney machines (600 ml/min).

c. Zirconium phosphate, a gel, breaks down during cation exchange, and phosphate and zirconium are released. The zirconium oxide serves, in part, to remove excess phosphate.

d. $H^+$ and $Na^+$ are released from zirconium phosphate, in exchange for $NH_4^+$. The amount of $H^+$ and $Na^+$ on the zirconium phosphate must exceed the expected $NH_4^+$ produced by urease, in order for the urease reaction to proceed to completion. Therefore, the initial pH surrounding the zirconium phosphate must be low, i.e. 6.1, and $H^+$ is added to the dialysate. This $H^+$ addition is in excess of the $HCO_3^-$ generated by the urease reaction. $H^+$ is toxic in kidney failure, as is $Na^+$.

e. Addition of $H^+$ to the dialysate must be countered by infusion of acetate, and by loading of the zirconium oxide with acetate.

f. Addition of $Na^+$ to the dialysate must be countered by allowing dilution in dialysate, with an initial $Na^+$ concentration lower than that of blood. About five l are required, prepared with vital electrolytes.

g. Zirconium phosphate, like most cation exchange materials, has a higher affinity for cations with higher charge density, such as $Ca^{++}$ and $Mg^{++}$, than for monovalent cations such as $NH_4^+$. $K^+$ is preferred to $Na^+$. Loading of the zirconium phosphate with $Ca^{++}$ would not allow $NH_4^+$ removal.

h. Excess removal of $Ca^{++}$, $Mg^{++}$, and $K^+$ must be countered by infusion of these cations, with the acetate, into the dialysate.

i. Use of about 5 l of dialysate requires that a heater still be present in the system.

j. As regeneration occurs at a distance from the membranes, adequate transfer of substances across the membranes requires a rapid water velocity near the membranes, as in standard dialysis equipment.

As a result of the above problems, presently developed systems of this type have tended to be complicated (in some cases more complicated than standard hemodialysis procedures), and the machine is relatively large.

Another cation exchanger that has been suggested for regeneration of dialysis fluid describes the possible use of zeolites for ammonium removal, with the same charcoal function as in the previously discussed system. This system includes:

a. A recycle system with zeolite as the $NH_4^+$ absorber, with the advantages of a high $NH_4^+/Na^+$ selectivity, and an exchange capacity of about 2mEq/gm (slightly higher than zirconium phosphate).

b. A system, especially developed with phillipsite as the $NH_4^+$ absorber.

c. A second zeolite to "control the pH" or exchange $H^+$ for $Na^+$, the $Na^+$ being added by the first zeolite in exchange for $NH_4^+$.

d. Urease, to split urea into $NH_4^+$ and $CO_3^=$.

Zeolites are aluminum silicates, with an alternating aluminum-oxygen-silicon-oxygen structure. They are crystals, in general, and do not decompose easily. The aluminum positions retain electro-positivity, and the silicon positions negativity.

Cation exchange occurs on the silicon position and because of the crystal structure, steric restrictions limit the size and shape of cations which exchange. Zeolites are among the few ion exchange materials which can select monovalent cations over divalent cations. The selectivities of these materials are such that the calcium and magnesium levels of the dialysis fluid may remain unchanged during the removal of $NH_4^+$.

In German patent 25.12212, issued to Gambro AG, it is suggested that the $NH_4^+$ exchange be performed on $Na^+$ loaded zeolite. A variety of natural zeolites are suggested for this exchange, in particular clinoptilolite and phillipsite. The selectives and functions of zeolites are thoroughly discussed by Donald Breck in his book *Zeolites: Molecular Sieves* published in 1972.

Several problems exist, however, with respect to zeolite usage in such systems.

a. When the system utilizes a column for regeneration of fluid, this would present problems with packing, maximal fluid flow, and separation of components with desirable interactions such as urease and cation exchanges.

b. Where a second zeolite is needed to remove the excess $Na^+$ generated by $NH_4^+$

exchange on the first zeolite, this zeolite would exchange $H^+$ for $Na^+$. The amount of $H^+$ on this zeolite would have to exceed the total amount of $NH_4^+$ expected to be generated during the splitting of urea. Thus, the $H^+$ would exceed the amount of $CO_3^=$, generated by usage, and the pH surrounding the $H^+$ loaded zeolite would be low during the entire reaction. If the initial pH is 6.4, for example, $H^+$ would thus be returned to the patient, and re-infusion of a base would be necessary.

c. Use of a second zeolite adds to the weight of zeolite needed. In the example set forth hereinafter, 1000 g of $Na^+$-loaded zeolite is coupled with 800 g of $H^+$-loaded zeolite.

d. When the system as described functions to maintain a *constant* concentration of calcium in the dialysate, calcium would not be added to the patient, and such calcium addition to a patient is desirable.

e. No provision has been hereafter made for the removal of phosphate.

f. A system for provision of rapid velocity of dialysate near the membranes of a dialyzer is needed, as the zeolites and other adsorbents are to be utilized at a distance from the membranes.

Thus, a need has existed for providing a dialysis composition and method that could overcome all, or at least most of, the foregoing problems.

Summary of the invention

This invention provides a novel mixture of compounds useful as a dialysis composition (i.e., as used herein a composition wherein dialysis occurs across a suitable membrane because of the presence of the composition to remove uremic substances (i.e., toxic substances that build up on kidney failure). The mixture of this invention, when positioned contiguous to one side of the membranes of an artificial kidney, removes uremic substances in blood at the other side of said membranes without the necessity of use of a water bath, a proportioning system, or a regenerating column as now used in known artificial kidneys. In addition, proper ion replacement is provided to the patient, particularly through the use of a mixture that includes a calcium-loaded zeolite cation exchanger suspended in a suspending agent.

It is the purpose of this invention to make available a dialysis composition in aqueous suspension form, for adsorption of uremic substances in an artificial kidney, comprising a surface active agent capable of adsorption of uremic substances and an enzyme to serve as a catalyst for urea degradation, characterized by comprising a suspending agent and a calcium-loaded zeolite exchanger. Such a composition makes possible hemodialysis without use of a water bath, a proportioning system regenerating column or a reinfusion of electrolytes. Such a material makes possible a smaller more compact artificial kidney.

The compositions of the invention are utilised by exposing said dialysis composition to blood so that said mixture adsorbs uremic substances from said blood to cleanse the same.

The accompanying drawings illustrate a complete embodiment of the invention according to the best mode so far devised for the practical application of the principles thereof, and in which:

Figure 1 is a cross-section view of an artificial kidney useful with dialysis composition of this invention; and

Figures 2 through 5 are graphs illustrating the invention.

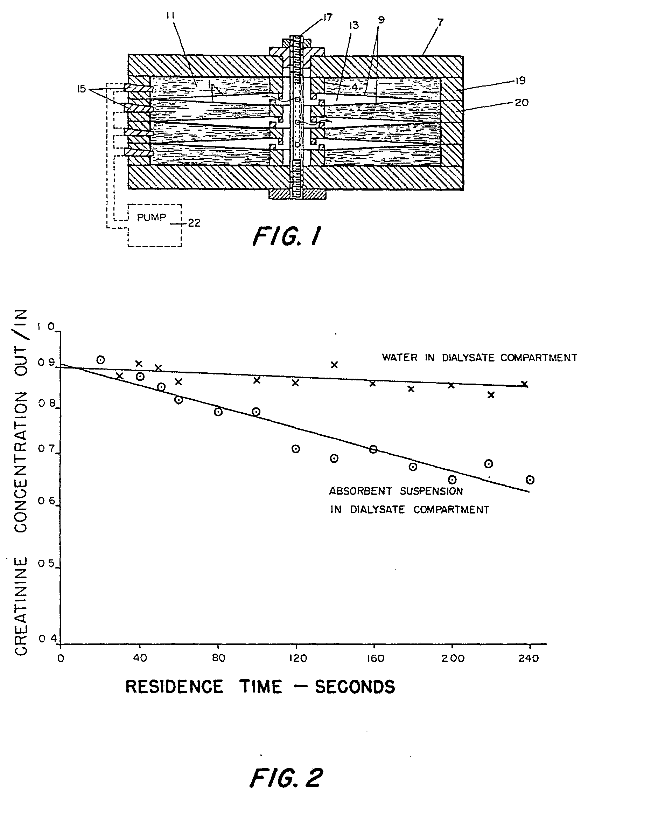

Referring now to the drawings, an artificial kidney 7 is illustrated in cross-section view of Figure 1. As shown, such a kidney normally includes a plurality of semi-permeable membranes 1 and 9 which are positioned so that dialysis composition 11 is at one side of the membrane and blood 13 is at the other side of the membrane, being separated by spacers 4. Suitable inlets 15 and 17 may be provided for the dialysis composition and blood, respectively. In a flow-through system, suitable outlets (not shown) would likewise be provided as is well known to one skilled in the art. The membranes (which could also be shaped as hollow fibers, for example, rather than as shown) may be clamped between clamps 19 and 20 or otherwise secured. Artificial kidney machines are well known and such machines are shown, by way of example, in the patents referred to hereinabove. Such apparatus has therefore been illustrated herein only so far as is necessary to better explain the invention.

An artificial kidney having a single blood inlet and outlet is described and claimed in U.S. Patent 4,071,444 by Stephen R. Ash, Philip G. Wilcox and David P. Kessler and entitled "Portable Chemical Reactor of Use as an artificial Kidney". This invention is also useful in such a device which is included herein by reference.

In this invention, effective removal of uremic substances through semi-permeable membranes 9 may be performed by the placement of a suspension of adsorbent compounds on the dialysis side of the membranes. The adsorbent compounds in this location substitutes for dialysate fluid which usually flows at a high velocity near the membranes, and also substitutes for the column used for the regeneration of dialysis fluid. Mass transfer is highly effective, as the diffusion distance for uremic substances in the dialysate side of the membrane is very small.

The following substances have distinct advantages for use in such an absorbent suspension:

a. Charcoal, or other surface absorptive agent,

b. Urease, in solution or suspension,

c. Zeolite cation exchangers, either naturally occurring or synthetic, which are loaded with calcium, and

d. A suspending agent, such as methylcellulose, hydroxyethyl starch or dextran.

This simple collection of adsorbents is capable of adsorbing all uremic substances, and furthermore, returning a beneficial ion flux to the patient, of calcium, and $HCO_3^=$. The suspension must move only fast enough to prevent saturation of the components near the membrane surface. This motion may be produced because of membrane motion in some dialyzer packages, or optionally by a small agitating pump 22 in other dialyzer packages (as indicated by the dotted pump insertion in Figure 1). In a hollow fiber configuration (not shown) with all of the adsorbents near a cellophane surface, it may be possible to effect dialysis with no movement of the adsorbent suspension.

While the zeolites shown tested herein are synthetic zeolites (Linde F-80 and W-85), phillipsite, and clinoptilolite, many other zeolite materials could be used. Alternate catalysts for the urea reaction are possible, besides urease. Charcoal could be replaced in the suspension by other surface adsorbent materials, such as an ion exchange resin e.g., Amberlite. Two or more cation exchangers could be added, and one of several suspending agents could also be utilized.

The placement of a suspension of adsorbing compounds next to dialysis membranes allows elimination of several components of standard dialysis machines such as the large water requirement, the mixture of ions and glucose with the water, water treatment, water heating systems, and high water velocity near the membranes (produced by narrow flow channels or high flow recirculating pumps). In addition, the suspension of adsorbents has advantages over the use of adsorbents in a column; and the column space requirements are avoided, and pumps and water connections from the dialysis membranes to the column are, of course, not needed. In addition, only a slow mixing of adsorbing compounds is necessary to prevent saturation of compounds near the membranes.

When adsorbent compounds are separated in layers, as in a column desirable interactions between the components are not possible. For example, urease is inhibited by its products $NH_4^+$ and $CO_3^=$ (through the development of $NH_3$ and high pH). $NH_4^+$ is bound by cation exchange materials. In a suspension of urease and cation exchangers, the progress of the urease reaction proceeds until the cation exchanger is saturated with $NH_4^+$. In a column with a partial separation of urease and cation exchangers, there is partial inhibition of urease.

When the adsorbent compounds are placed next to dialysis membranes then the distance necessary for diffusion of uremic substances is small before removal from solution. Excellent mass transfer occurs across the membranes because the uremic substances are kept in low concentration in the dialysis compartment. Thus, a suspension of adsorbing compounds, as used in this invention, has good effectiveness even at slow flow of the suspension and its effectiveness at slow flow is better than that of water at high flow velocity.

Figure 2 indicates the time course of removal of one uremic substance creatinine, through cellulose membranes. The vertical axis is the ratio of creatinine concentration in fluid leaving the membrane packages to the "in" flow concentration. This membrane package utilizes an "in and out" flow of fluid, and therefore, the time of residence of the creatinine solution in the packages is expressed in "cycle time".

The lower curve represents concentration in the absorbent suspension in the dialysate compartment and is characterised by the expression

$$C/C_0 = 0.9111_e{}^{-.00158t},$$

with a correlation coefficient $r^2 = 0.9086$, and the upper curve represents concentration in water in the dialysate compartment characterised by the expression

$$C/C_0 = 0.8985_e{}^{-.00024t},$$

with a correlation coefficient, $r^2 = 0.439$.

It is seen that a much more effective removal of creatinine occurred when membranes were surrounded by a "slurry" or suspension of adsorbing compounds, than when the membranes were surrounded by water. Even when water flow was very high in this dialyzer membrane package, the effectiveness of removal of creatinine (mass transfer coefficient) does not reach that when the slurry was utilized.

When adsorbing compounds are placed in a column, an increase of resistance occurs when flow rates increase. This phenomenon is called "packing". Accompanying this packing is "channeling", or development of flow pathways which avoid much of the adsorbent compounds. As a consequence, the capacity of columns rarely equals that of adsorbing compounds in a suspension. In fact, the maximal capacity of adsorbing compounds is usually measured in a suspension. The use of suspension of adsorbents avoids this problem.

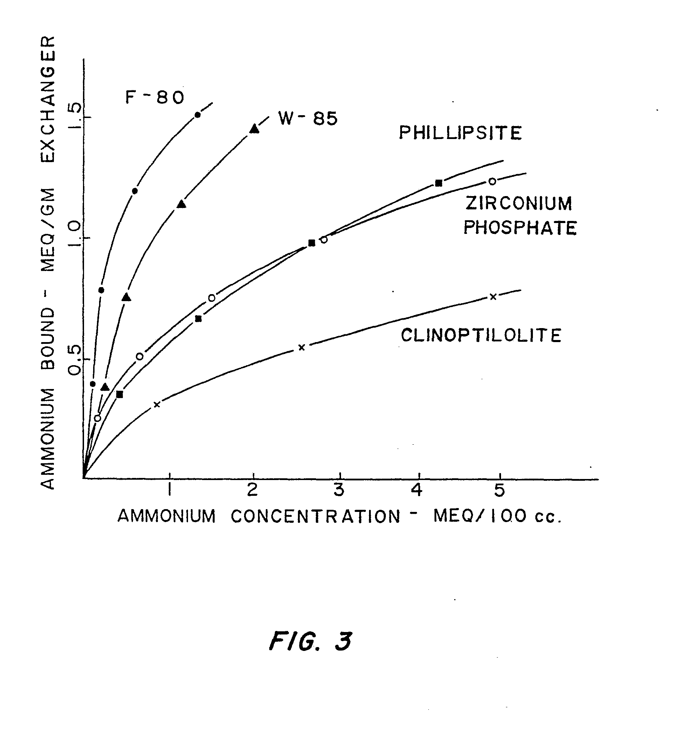

The selectivity of zeolites for ammonium is significantly higher than most other cation exchange materials. Furthermore, the total (maximal) exchangeable cations is higher for zeolites than most exchangers (7 mEq/gm for a synthetic zeolite (Linde F-80), according to Union Carbide technical data, vs. 2.0 mEq/gm for zirconium phosphate, according to CCI data). As a result, the removal of ammonium is higher, for each supernatant ammonium concentration, for zeolites. Figure 3 indicates the amount of ammonium bound on various cation exchangers, during titration of a suspension of these exchangers with NH$_4$Cl.

The horizontal axis of the graph indicates the ammonium concentration of the supernatant at equilibrium and the amount of ammonium bound, vertical axis, is determined by subtraction of the ammonium content of the supernatant from the amount added to the suspension.

All of the exchangers are Na$^+$ loaded, with the exception of zirconium phosphate, which is H$^+$—Na$^+$ loaded. As is indicated, synthetic zeolites (Linde F-80 and W-85) bind approximately twice the NH$_4^+$, at a low concentration of NH$_4^+$, as zirconium phosphate or phillipsite. Zeolite binds approximately 4 times the NH$_4^+$ of clinoptilolite, at each NH$_4^+$ concentration.

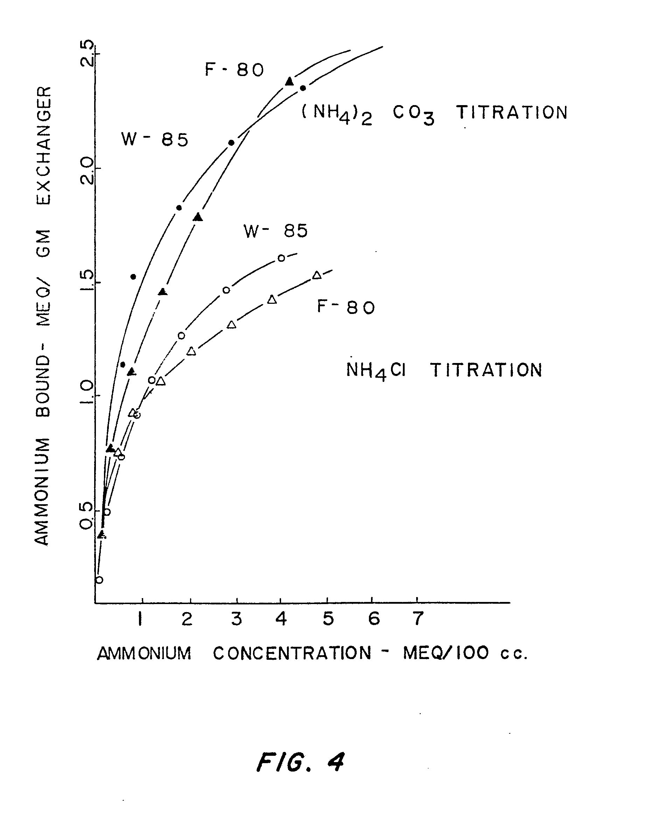

The crystalline framework of zeolite cation exchangers tends to exclude ions with certain sizes and charge densities. As a result, zeolite materials are among the few cation exchange materials which have a higher selectivity for ammonium than for divalent cations such as calcium. This property allows the loading of zeolites with calcium and subsequent exchange with ammonium. This exchange has been used for decades in the purification of water in waste treatment. Figure 4 shows the results of experiments in which zeolites were treated with ammonium chloride and ammonium carbonate and indicates the binding of ammonium on synthetic zeolites (Linde F-80 and W-85), after calcium loading of these materials (50% calcium, 50% sodium). The amount of ammonium bound was calculated by subtraction of the ammonium content of the supernatant from the amount added to the suspension and the horizontal axis shows the ammonium concentration of the supernatant at equilibrium.

The lower curves indicate titration of the zeolites with NH$_4$Cl. Comparison with a similar curve in Figure 3 indicates that the binding of ammonium is only slightly decreased by calcium loading of zeolites, vs. sodium loading of zeolites. In contrast, the loading of zirconium phosphate with calcium allows almost no exchange of ammonium for calcium, due to a high selectivity of zirconium phosphate for calcium.

Figure 4 also indicates the titration of synthetic zeolites with ammonium carbonate. As indicated, the binding of ammonium at the higher pH levels caused by the ammonium carbonate, is significantly greater than the binding at the lower pH levels during ammonium chloride titration. Since the product of urease is ammonium carbonate the titration of zeolite with ammonium carbonate should predict the binding of ammonium in the presence of urea and urease.

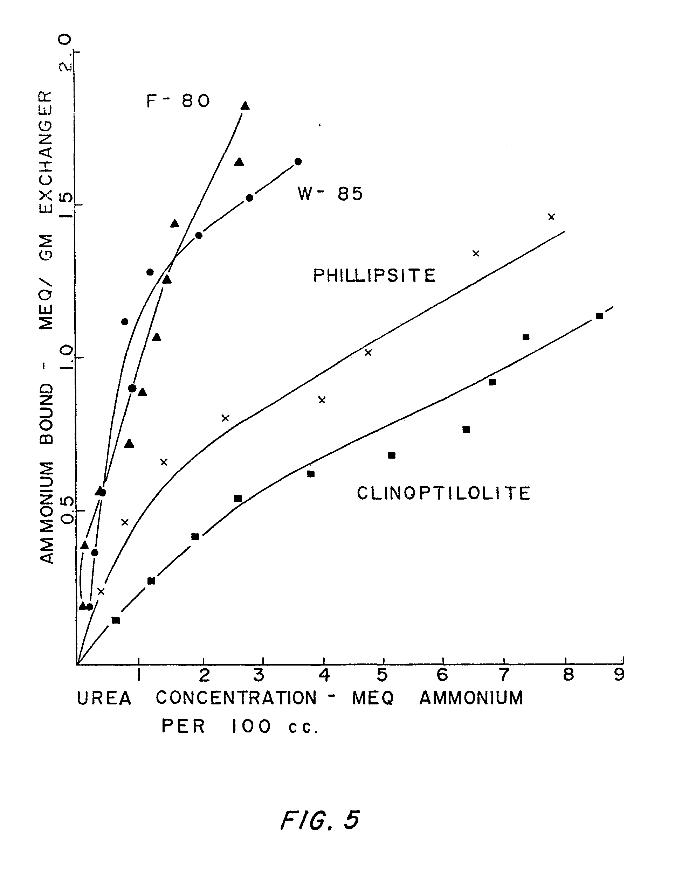

Because zeolites have selectivities which allow exchange of ammonium for calcium, and because of the optimal binding at high pH levels, calcium loaded zeolites remove nitrogen well during the urease reaction. Figure 5 indicates the ammonium binding on calcium loaded zeolites after addition of urea to the supernatant. The supernatant concentration of urea is expressed as ammonium (two ammonium ions developed per urea molecule). Loading is performed by twice suspending zeolite in 0.4 m CaCl$_2$, twice centrifuging, and resuspending in water. Urease is present in low concentration, 0.5 g or 200 Sumner units per 100 cc, and 30 minutes is allowed for completion of the reaction. The results indicate that the binding of ammonium is approximately the same as for titration of zeolites with ammonium carbonate; at a concentration of 2 mEq ammonium (from urea), per 100 cc, in the supernatant, 1.4 mEq ammonium is bound per gram of synthetic zeolites (Linde F-80 and W-85) (calcium loaded).

It is important, of course, to remove urea at a rate which exceeds the transfer of urea across dialysis membranes. Thus, the equilibrium ammonium binding of zeolite is not as relevant as rapidity of binding during the urease reaction. In the presence of 10 gm of cation exchanger per 100 cc, urease operates at a maximal velocity equivalent to the Sumner units determined by the manufacturer. Excess urease (5 gm/100 cc) allows binding of urea similar to equilibrium binding (Figure 5) in two minutes.

The density of zeolite materials is much less than that for zirconium phosphate (specific gravity of 2.5). This allows greater ease of suspension, important for the maintenance of adsorbing compounds near the surface of membranes. In a solution of 0.5% methylcellulose, 1500 centipoise, all zeolites tested have been suspended indefinitely synthetic zeolites (Linde F-80 and W-85), phillipaite, and clinoptilolite). This zeolite suspension is also persistent after the addition of charcoal and urease. Zirconium phosphate gel (CCI Life Systems) is suspended only 10 minutes by this concentration of methylcellulose.

Prolonged particle suspension of adsorbing compounds allows these compounds to be kept in juxtaposition to dialysis membranes without a rapid flow rate of the suspension. A slow flow of the suspension is sufficient to prevent adsorbing compounds at the membrane surface from being saturated.

In the device, shown in U.S. Patent 4,071,444, movement of membranes, due to blood compartment volume change, is adequate to mix the adsorbing compounds suspension and prevent saturation. In other dialyzers, the flow of suspension need move the total suspension volume through the membrane package only 2 to 3 times during each treatment.

The crystalline nature of zeolites produces more stability than that of gel materials. Zirconium phosphate, a gel, dissolves to a slight degree during cation exchange. The resultant phosphate is bound by zirconium oxide in one prior art system column. Zeolites do not dissolve during exchange reactions.

Figures 2—4 indicate that zeolites loaded with calcium can effectively remove ammonium during the urease reaction. However, zeolites loaded with sodium, as depicted in Figure 3, would seem to work equally well. Several distinct advantages attend the use of calcium-loaded zeolite materials in the suspension:

1. Calcium is released during the exchange for ammonium, and some of this calcium returns to the patient through the dialysis membranes. This calcium is beneficial to the patient, as it replaces the body stores of calcium, and decreases the level of parathyroid hormone, usually high in dialysis patients. Calcium is one of the few cations *not* toxic in kidney failure. Sodium, hydrogen, potassium, and magnesium are all potentially toxic.

2. $CO_3^=$ is developed during the urease reaction. This is not countered by $H^+$ on the cation exchange material (as with $H^+$—$Na^+$ loaded zirconium phosphate or zeolite). Therefore, some of this $CO_3^=$ is free to return to the patient. As patients with kidney failure are acidotic, this $CO_3^=$ is beneficial to the patients. For example, $CO_3^=$ causes removal of $H^+$ from bones, and allows more deposition of calcium. Parathyroid hormone is also decreased if acidosis is corrected.

3. Neither calcium nor $CO_3^=$ is returned to the patient in great excess, or in amounts equal to the amount of urea removed. This is because the precipitation of calcium carbonate on the dialysis side of the membrane prevents passage through dialysis membranes into the bloodstream of the patient. During *in vitro* tests of the urease reaction in the presence of calcium-loaded zeolites the calcium in the supernatant actually falls. For example, the binding of 2 mEq/gm of ammonium during this reaction is associated with a fall in supernatant calcium from 27 mM/L to 1 mM/L. This can be explained by the precipitation of calcium and bicarbonate. The bicarbonate returned to the patient is also reduced by this precipitation. If 18 grams of urea is produced by a patient daily, for example, 18 grams or 300 mM must be removed. This is equivalent to 600 mEq of ammonium generated during the urease degradation. The return of 600 mEq of calcium, or 600 mEq of $CO_3^=$ to the patient would be excessive. Therefore, it is fortuitous that precipitation of calcium and bicarbonate limits the return of these substances through the dialysis membrane.

4. Calcium and phosphate also precipitate on the dialysis side of the membrane. Since phosphate must be removed during the dialysis procedure, this precipitation is beneficial, as it results in decrease in blood phosphate levels. A specific phosphate binder, as zirconium oxide for example, is not necessary. A recent experiment with a uremic dog indicated that the phosphate removal through a membrane package was such that the blood concentration dropped 50% between in-flow and out-flow blood. This was approximately the same drop in concentration as for creatinine, urea, and potassium.

5. Sodium loading of zeolites, or hydrogen loading, results in return to the patient of sodium or hydrogen, both of which are toxic substances in renal failure. Reinfusion of base, and 5 l of dialysate are necessary in some prior art systems. Reinfusion of base is necessary to prevent this return of $H^+$, and 5 l of fluid with low Na concentration is necessary for dilution of Na released. Use of calcium-loaded zeolites eliminates the necessity for re-infusion of base or large dialysate volumes.

6. Only one exchange material is necessary, in comparison to two in other prior art systems. Thus weight and bulk of absorbents is decreased. The preferred zeolite is loaded with 30—80% calcium, 20—50% sodium and 0—20% potassium on a molar basis.

In summary, placement of a suspension of adsorbents next to the membranes of an artificial kidney offers the advantage of making the dialysis equipment simpler, and of increasing mass transfer through the membranes. Zeolite materials are well suited to the absorption of ammonium, produced by the urease reaction with bicarbonate. The loading of such zeolite materials with calcium results in beneficial calcium and carbonate fluxes to the patient. The calcium also limits the return of phosphate to the patient. The fluxes of calcium and carbonate are less than the flux of urea out of the animal. This is due to precipitation of calcium, phosphate and calcium-carbonate in the dialysis chamber. Sodium and hydrogen overloading of the patient is avoided, a problem with other regenerative systems.

**Claims**

1. A dialysis composition in aqueous suspension form, for adsorption of uremic substances in an artificial kidney, comprising a surface adsorptive agent capable of adsorption of uremic substances and an enzyme to serve as a catalyst for urea degradation, characterized by comprising a calcium-loaded zeolite exchanger and a suspending agent.

2. A dialysis composition according to claim 1 wherein the surface adsorptive agent comprises charcoal.

3. A dialysis composition according to either of claims 1 and 2 wherein the surface adsorptive agent comprises an ion exchange resin.

4. A dialysis composition according to any of the preceding claims wherein the enzyme is urease.

5. A dialysis composition according to any of the preceding claims wherein the suspending agent is methylcellulose, hydroxyethyl starch or dextran.

6. A dialysis composition according to any of the preceding claims wherein the zeolite exchanger is one of phillipsite, clinoptilolite and synthetic zeolites.

7. A dialysis composition according to any of the preceding claims wherein the exchanger is loaded with 30 to 80 percent calcium, 20 to 50 percent sodium and 0 to 20 percent potassium on a molar basis.

## Patentansprüche

1. Dialysepräparat in Form einer wässrigen Suspension zur Adsorption von urämischen Substanzen in einer künstlichen Niere, enthaltend ein Oberflächenadsorptionsmittel zur Adsorption urämischer Substanzen und ein als Katalysator für den Harnstoffabbau dienendes Enzym, dadurch gekennzeichnet, daß es einen Calcium-beladenen Zeolith-Austauscher und ein Suspendierungsmittel enthält.

2. Dialysepräparat nach Anspruch 1, dadurch gekennzeichnet, daß das Oberflächenadsorptionsmittel Holzkohle ist.

3. Dialysepräparat nach Ansprüchen 1 oder 2, dadurch gekennzeichnet, daß das Oberflächenadsorptionsmittel ein Ionenaustauscherharz ist.

4. Dialysepräparat nach einem der Ansprüche 1 bis 3, dadurch gekennzeichnet, daß das Enzym Urease ist.

5. Dialysepräparat nach einem der Ansprüche 1 bis 4, dadurch gekennzeichnet, daß das Suspendierungsmittel Methylcellulose, Hydroxyethyl-Stärke oder Dextran ist.

6. Dialysepräparat nach einem der Ansprüche 1 bis 5, dadurch gekennzeichnet, daß der Zeolith-Austauscher aus der Gruppe der Phillipsite, Clinoptilolite und synthetischen Zeolithe ist.

7. Dialysepräparat nach einem der Ansprüche 1 bis 6, dadurch gekennzeichnet, daß der Austauscher mit—auf molarer Basis—30 bis 80% Calcium, 20 bis 50% Natrium und 0 bis 20% Kalium beladen ist.

## Revendications

1. Composition de dialyse sous forme de suspension aqueuse, pour l'adsorption de substances urémiques dans un rein artificiel, comprenant un agent adsorbant de surface capable d'absorber des substances urémiques et une enzyme pour servir de catalyseur pour la dégradation de l'urée, caractérisée en ce qu'elle comprend un échangeur à base de zéolite chargée de calcium et un agent de mise en suspension.

2. Composition de dialyse selon la revendication 1, caractérisée en ce que l'agent adsorbant de surface comprend du charbon de bois.

3. Composition de dialyse selon la revendication 1 ou la revendication 2, caractérisé en ce que l'agent adsorbant de surface comprend une résine échangeuse d'ions.

4. Composition de dialyse selon l'une quelconque des revendications précédentes, caractérisée en ce que l'enzyme est l'uréase.

5. Composition de dialyse selon l'une quelconque des revendications précédentes, caractérisée en ce que l'agent de mise en suspension est la méthylcellulose, l'hydroxyéthylamidon ou le dextrane.

6. Composition de dialyse selon l'une quelconque des revendications précédentes, caractérisée en ce que l'échangeur à base de zéolite est choisi parmi la phillipsite, la clinoptilolite et des zéolites de synthèse.

7. Composition de dialyse selon l'une quelconque des revendication précédentes, caractérisée en ce que l'échangeur est chargé avec 30 à 80% de calcium, 20 à 50% de sodium et 0 à 20% de potassium sur une base molaire.

FIG. 1

FIG. 2

FIG. 3

FIG. 4

## 0 003 914

*FIG. 5*

4